# EUROPEAN PATENT APPLICATION

(11) **EP 4 786 573 A1**
(43) Date of publication of application: **05.08.2026**
(21) Application number: 25155221.2
(22) Date of filing: 31.01.2025
(51) Int. Cl.: C12M 1/42, C12M 1/00, C12M 1/34, C12M 1/36

(54) **MICROELECTRODE ARRAY TO DELIVER CHARGED ENTITIES IN CELLS THROUGH ELECTROPORATION**

(71) Applicant: Imec VZW, 3001 Leuven (BE)
(72) Inventor: DUCKERT, Bastien, 3001 Heverlee (BE); ZHANG, Lei, 3010 Kessel-Lo (BE); HU, Xuqu, 3001 Leuven (BE); MARCHAL, Elisabeth, 3052 Blanden (BE)
(74) Representative: Winger

(57) **Abstract**

A device for delivering charged entities into biological cells of a biological cellular material, comprising a microelectrode array for supporting the biological cellular material, the microelectrode array comprising individually addressable electrodes made of an electrically-conductive CMOS-compatible material having an oxide top layer, and the charged entities electrostatically coupled to the oxide top layer. The oxide top layer may be a native oxide top layer. The charged entities can be negatively charged entities electrostatically attached to positively charged carriers directly attached to the oxide top layer, or positively charged entities encapsulated in a negatively charged carrier electrostatically coupled to the native oxide top layer. The electrically-conductive CMOS-compatible material can be a metal, metal nitride, or metal oxide. Charged entities differing in their characteristics may be electrostatically coupled to distinct electrodes of the microelectrode.

## Description

### Field of the Invention

The present invention relates to the field of screening of biologically active entities, and more in particular to devices and methods for enabling such screening.

### Background of the Invention

Nucleic acid delivery into cells is a fundamental technique in molecular biology and biotechnology. It enables the introduction of foreign genetic material into cells for various applications, including gene therapy, functional genomics, and drug discovery. Traditional methods for nucleic acid delivery have relied on chemical transfection reagents or bulk electroporation techniques.

Electroporation is typically performed in bulk, where high voltage pulses are applied to cell suspensions in a cuvette to temporarily permeabilize cell membranes, allowing nucleic acids to enter. The cuvette contains two electrode plates across which a substantial electrical potential is applied.

In traditional cuvette electroporation, each nucleic acid solution and cell suspension mixture must be processed in separate cuvettes, each equipped with its own pair of electrode plates. This requirement limits the potential for miniaturization and scaling up the screening of molecules. Additionally, cuvette electroporation demands a large number of cells, significant volumes of nucleic acid solution, and considerable manual effort.

Furthermore, this method often results in significant cell death due to the high voltages used and necessitates large quantities of cells and nucleic acids.

These requirements hinder its suitability for screening large numbers of distinct nucleic acids efficiently.

Chemical transfection, also called reversed transfection, involves complexing nucleic acids with a chemical transfection reagent (e.g., cationic lipids) to facilitate cellular uptake. When cells are dispensed in bulk grown on the surface on which the complexes are present, the solubilization of the transfection mix in the cell culture medium triggers the transfection of the cells. While this widely used concept can allow for higher throughput screenings than bulk cuvette electroporation, chemical transfection can suffer from low transfection efficiency, high cytotoxicity, and limited applicability across different cell types.

There is an ongoing need for improved methods to deliver nucleic acids into cells with high efficiency and low toxicity. In particular, the ability to screen large libraries of nucleic acid constructs in a high-throughput manner remains challenging with existing techniques. Current approaches like reverse transfection arrays allow parallel testing of multiple constructs, but are limited by the low efficiency of chemical transfection methods.

Improved screening methods would accelerate many areas of biomedical research and development. Applications could include rapid screening of gene therapies, optimization of CRISPR gene editing components, and discovery of novel RNA therapeutics. Despite progress in the field, there remains a need for devices and methods overcoming at least partially one or more of the above issues.

### Summary of the Invention

It is an object of embodiments of the present invention to enable screening of charged entities delivered into cells. This objective is accomplished by a device and a method for delivering charged entities through electroporation into biological cells according to the invention.

In the first aspect, the present invention relates to a device for delivering charged entities through electroporation into biological cells of a biological cellular material, comprising:
- a microelectrode array for supporting the biological cellular material, the microelectrode array comprising individually addressable electrodes made of an electrically-conductive CMOS-compatible material having an oxide top layer, and
- the charged entities electrostatically coupled to the oxide top layer.

This allows for a stable, yet reversible, controlled immobilization of the charged entities. This prevent mixing of entities with different characteristics.
In embodiments, the oxide top layer may be a native oxide top layer. This allows for direct electrostatic coupling without additional processing.

In embodiments, the charged entities may be negatively charged entities electrostatically attached to positively charged carriers, e.g., molecules, themselves directly attached to the oxide top layer. This enables coupling of negatively charged entities.

In embodiments, the charged entities may be positively charged entities, e.g., encapsulated in a negatively charged carrier electrostatically coupled to the native oxide top layer. This enables coupling of positively charged entities.

In embodiments, the electrically-conductive CMOS-compatible material may be a metal, a metal nitride or oxide, preferably a metal or a metal nitride. Examples of metal oxides are iridium oxide and ruthenium oxide. These materials are compatible with standard CMOS processing.

In embodiments, the CMOS-compatible material may be selected from the group consisting of Titanium Nitride, Ruthenium, Platinum, and Iridium. These are preferred CMOS-compatible electrode materials.

In embodiments, the positively charged carriers may be polymers, preferably branched polymers, for instance Poly(ethylene-imine) (PEI) molecules or poly-lysine. These polymers provide multiple positive charges for coupling.

In embodiments, charged entities differing in their characteristics may be electrostatically coupled to distinct electrodes of the microelectrode array. This allows screening multiple entities in parallel.

In embodiments, the negatively-charged entities may be nucleic acids or polypeptides such as proteins. These are common biomolecules of interest for screening.

In embodiments, the nucleic acids may be selected from the group consisting of DNA and RNA, and more preferably guide RNAs for CRISPR-Cas9. This enables screening for gene editing.

In the second aspect, the present invention relates to a method for delivering charged entities through electroporation into biological cells of a biological cellular material, comprising:
- a. Providing a device according to any embodiments of the first aspect,
- b. Providing a biological cellular material over the microelectrode array,
- c. Applying an electrical field to one or more of said electrodes to which charged entities are electrostatically coupled so as to release said charged entities from the top layer and allow said charged entities to be delivered into said biological cells of said biological cellular material.

In embodiments, step c may cause an effect in said biological cells, and the method may further comprise a step d. of detecting said effect. This allows determining the impact of the delivered entities.

In embodiments, in step a. said charged entities may differ in their characteristics and be electrostatically coupled to distinct electrodes of the microelectrode array. This enables screening multiple entities in parallel.

In embodiments, said biological cellular material may be a cell culture or a tissue. The method is applicable to various biological samples.

In embodiments, the method may further comprise creating a dataset comprising said characteristics of said charged entities associated with the effects detected for said charged entities. This dataset correlates entities and their cellular effects.

In embodiments, the method may further comprise analyzing the dataset, e.g. using a machine learning algorithm, to correlate said effects with characteristics of the entities associated with said effect. This analysis identifies structure-function relationships.

In embodiments, the method may further comprise designing new entities based on the analysis of the dataset. The structure-function relationships guide rational design of new entities.

In embodiments, the method may further comprise repeating steps a to c or a to d while using the new entities in step a. This enables iterative optimization of the entities.

In embodiments, the electrical field may be individually applied to each of said electrodes in a controlled manner, taking into account one or more cellular health factors, so as to enable electroporation while preventing cellular death directly caused by the electrical field. This maximizes cell viability.

In embodiments, the method may further comprise measuring the permeability of the cell membrane above each electrode, and adjusting the applied electrical field based on the measured permeability of the cell membrane. This provides feedback control to optimize electroporation.

In embodiments, the effect on the biological cells may be a change in cell morphology, viability, or gene expression. These are relevant cellular readouts.

In embodiments, step a may comprise first a1. coupling electrostatically said charged entities on different electrodes of the multielectrode array, then a2. selectively removing some of said entities by applying an electrical field to some of the electrodes, then a3. providing the biological cellular material over the multielectrode array. This allows patterning the entities at higher resolution than the initial deposition.

In embodiments, before step a, the multielectrode array may be cleaned by UV-ozone and/or by a wet cleaning, e.g. a wet oxidative cleaning. This prepares the surface for electrostatic coupling.

In embodiments, step b may provide the biological cellular material in physical contact with said charged entities. This is preferred but not mandatory as the biological cellular material may instead be suspended over the microelectrode array without physical contact with said charged entities and without physical contact with the microelectrode array.

In embodiments, step b may provide the biological cellular material in physical contact with said charged entities and with said multielectrode array. This is the ideal case.

In embodiments where the biological cellular material is suspended over the microelectrode array without physical contact with said charged entities and without physical contact with the microelectrode array, the biological cellular material is preferably sufficiently close to the charged entities so that at least some degree of spatial selectivity is maintained.

In embodiments, the biological cellular material may be provided in step b in suspension above the microelectrode array without physical contact with the microelectrode array. This reduces mechanical stress on the cells.

In embodiments, step b may comprise introducing the biological cellular material in suspension into a chamber immediately before performing step c. This enables rapid processing without waiting for cell adhesion.

In embodiments, the device may comprise a porous membrane positioned above the microelectrode array surface, and step b may comprise allowing the biological cellular material to settle onto said porous membrane while remaining suspended above the electrode array before performing step c. This provides controlled positioning of suspended cells.

In embodiments, step c may be performed while the cells are in suspension above the electrodes. This provides high cell viability.

In the third aspect, the present invention relates to a system comprising the device of any embodiments of the first aspect and further comprising means adapted to execute one or more steps, preferably all steps, of the method of any embodiments of the second aspect.

In embodiments, said means may comprise a delivery unit for delivering coupling means over said microelectrode array and/or for delivering said charged entities over said microelectrode array so that charged entities differing in their characteristics are coupled to different electrodes by coupling means. This automates the entity deposition step.

In embodiments, the delivery unit may comprise an arraying tool, e.g., a spotting tool. This enables precise spatial patterning of the entities.

In embodiments, the means may comprise a detector for detecting the effect on the biological cells of the electroporation. This automates the readout step.

In embodiments, the detector may comprise an imaging unit, such as a fluorescence imaging unit and/or a lens-free imaging unit. Imaging enables spatial mapping of the effects.

In embodiments, the means may comprise a control unit configured for performing one or more, and preferably all of the steps of operating the delivery unit, applying the electrical field, controlling the electrical field, measuring cell permeability, creating the dataset, analyzing the dataset, designing new entities, and iterating the process. The control unit automates the entire workflow.

In embodiments, the control unit may be configured for analyzing the dataset, e.g. using a machine learning algorithm, to correlate said effects with characteristics of the entities associated with said effect, and the system may further comprise means for designing new entities based on said correlation. This enables a closed-loop optimization process.

In the fourth aspect, the present invention relates to a computer program comprising instructions to cause the system of any embodiments of the third aspect to execute the steps of the method of any embodiments of the second aspect.

In the fifth aspect, the present invention relates to a computer-readable medium having stored thereon the computer program of the fourth aspect.

In the sixth aspect, the present invention relates to a kit for electroporating charged entities into biological cells of a biological cellular material, comprising:
- a. A microelectrode array for supporting the biological cellular material, the microelectrode array comprising individually addressable electrodes made of an electrically-conductive CMOS-compatible material having an oxide top layer,
- b. coupling means for electrostatically coupling charged entities to said electrodes.

In embodiments, the oxide top layer may be a native oxide top layer. This provides the components for implementing the invention.

In embodiments, the kit may further comprise said charged entities. This provides a complete ready-to-use kit.

It is an advantage of embodiments of the present invention that a device for electroporating charged entities into biological cells can be achieved using a microelectrode array with individually addressable electrodes made of an electrically-conductive CMOS-compatible material.

It is a further advantage of embodiments of the present invention that the oxide top layer can be a native oxide top layer, simplifying the device fabrication process.

It is a further advantage of embodiments of the present invention that charged entities differing in their characteristics can be electrostatically coupled to distinct electrodes of the microelectrode array, preventing their mixing, and enabling multiplexed screening applications.

It is another advantage of embodiments of the present invention that the method can further include detecting an effect caused in the biological cells and creating a dataset correlating the characteristics of the charged entities with the detected effects for analysis and design of new optimized entities.

It is a further advantage of embodiments of the present invention that a system can be provided to execute the steps of the electroporation and screening method in an automated manner.

It is an advantage of embodiments of the present invention that a high-throughput screening of entities delivered into cells can be achieved.

It is a further advantage of embodiments of the present invention that different entities can be pre-arrayed on a high-density microelectrode array designed for electroporation of cells grown atop its electrodes.

It is an advantage of embodiments of the present invention that entities can be released from the chip and delivered into cells upon submission of an electrical field.

It is a further advantage of embodiments of the present invention that a largely automated and plug-and-play operation can be implemented.

It is an advantage of embodiments of the present invention that tens of thousands of different entities can be screened within a single week.

It is a further advantage of embodiments of the present invention that high delivery efficiencies and low cytotoxicity can be achieved compared to chemical transfection or bulk electroporation.

It is an advantage of embodiments of the present invention that feedback control of the cell delivery process can be implemented by measuring cell membrane permeability.

It is an advantage of embodiments of the present invention that spatial selectivity in charged entity delivery can be achieved through the use of individually addressable microelectrodes.

It is a further advantage of embodiments of the present invention that the workflow can be simplified for the user with minimal handling steps required.

Particular and preferred aspects of the invention are set out in the accompanying independent and dependent claims. Features from the dependent claims may be combined with features of the independent claims and with features of other dependent claims as appropriate and not merely as explicitly set out in the claims.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

Fig. 1 is a flowchart of the method for high throughput screening of nucleic acids based on charged entity electrotransfer according to embodiments of the present invention.
Fig. 2 is a schematic diagram of the system for screening of charged entities based on charged entity electrotransfer according to embodiments of the present invention.
Fig. 3 is an illustration of a transfection screening workflow based on DNA immobilization on a microelectrode array (MEA) according to embodiments of the present invention.
Fig. 4 is a schematic diagram of plasmid DNA immobilization on the native oxide layer of a Ruthenium or Titanium Nitride electrode according to embodiments of the present invention.
Fig. 5 is an illustration of the automated and plug-and-play operation of the system for high throughput screening of nucleic acids based on gene electrotransfer according to embodiments of the present invention.

In the different figures, the same reference signs refer to the same or analogous elements and dashed lines represent optional features.

### Detailed description of Illustrative Embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

The following terms are provided solely to aid in the understanding of the invention.

As used herein, and unless otherwise specified, the term *"entities"* refers to any discrete units or substances that can be applied to or interact with biological cells, including but not limited to molecules, particles, or organisms. Examples of entities include entities, proteins, small molecules, nanoparticles, viruses, or bacteria.

As used herein, and unless otherwise specified, the term *"charged entities (10)"* refers to entities that carry an electric charge, such as negatively charged entities or positively charged entities. Examples of charged entities include nucleic acids (e.g., DNA, RNA, guide RNAs for CRISPR-Cas9), polypeptides (e.g., proteins), and charged carriers encapsulating or attached to other entities.

As used herein, and unless otherwise specified, the phrase "delivering charged entities through electroporation into biological cells" refers to the application of an electrical field that facilitates the entry of the charged entities into the interior of the biological cells. This process may involve temporarily altering the permeability of the cell membrane to allow the passage of the charged entities from the extracellular environment into the cytoplasm or other intracellular compartments. The delivery method may be designed to be efficient while minimizing damage to the cells. The delivery process may be controlled and optimized to ensure that the entities can cross the cell membrane barrier and reach the intracellular space where they can exert their biological effects. This may involve one or more of the following: the control of the strength of the applied electrical field, the control of the duration of the applied electrical field, and the control of the timing of entity release from the substrate.

As used herein, and unless otherwise specified, the term "biological cells (20)" refers to cells that are derived from living organisms and are capable of carrying out biological functions. Examples of biological cells include cells in cell cultures, cells in tissues, and cells in organs.

As used herein, and unless otherwise specified, the term *"biological cellular material"* refers to any collection of biological cells, whether in a monolayer or three-dimensional structure. Examples of biological cellular material include cell cultures, tissues, organoids.

As used herein, and unless otherwise specified, the term *"multielectrode array"* refers to a device comprising multiple individually addressable electrodes arranged in a pattern on a substrate. Examples of multielectrode arrays include planar electrode arrays, 3D electrode arrays, or microelectrode arrays integrated with CMOS circuitry.

As used herein, and unless otherwise specified, the phrase *"for supporting the biological cellular material"* refers to the capability of the microelectrode array to provide a surface or substrate on which the biological cellular material can be placed, adhered, or cultured. This support function allows the biological cellular material to be in close proximity to or in direct contact with the electrodes of the microelectrode array, enabling effective electroporation and delivery of charged entities into the biological cells.

As used herein, and unless otherwise specified, the term *"individually addressable electrodes"* refers to electrodes in a microelectrode array that can be independently controlled and activated. Each electrode can receive distinct electrical signals or pulses, allowing for precise spatial and temporal control over the electroporation process. This individual addressability enables the selective activation of specific electrodes, facilitating the targeted delivery of charged entities into biological cells at specific locations on the microelectrode array. The ability to control each electrode independently also allows for optimized electroporation parameters for different areas of the biological cellular material, potentially improving the efficiency and specificity of the delivery process.

As used herein, and unless otherwise specified, the term *"CMOS-compatible material"* refers to materials that can be used in the fabrication of complementary metal-oxide-semiconductor (CMOS) devices without contaminating or interfering with the CMOS manufacturing process. As used herein, and unless otherwise specified, the term "electrically-conductive CMOS-compatible material (160)" refers to a material that is electrically conductive and compatible with complementary metal-oxide-semiconductor (CMOS) fabrication processes. Examples of electrically-conductive CMOS-compatible materials include metals (e.g., titanium nitride, ruthenium, platinum, iridium), metal nitrides, and metal oxides (e.g., iridium oxide, ruthenium oxide).

As used herein, and unless otherwise specified, the term "oxide top layer (170)" refers to a layer of oxide material that is present on the surface of the electrically-conductive CMOS-compatible material. In embodiments, the oxide top layer may be a native oxide layer that forms naturally on the surface of the material.

As used herein, and unless otherwise specified, the term "electrostatically coupled" refers to the non-covalent attachment of charged entities to a surface through electrostatic interactions, which can occur either directly or indirectly. In the context of this invention, it describes the association between the charged entities (such as nucleic acids or other biomolecules) and the oxide top layer of the electrodes. This electrostatic coupling can be:
Direct: where the charged entities are held in place by direct electrical forces exerted by the charged surface of the oxide layer. This is more likely the case when the charged entities are positively charged.
Indirect: where the charged entities are attached to intermediary molecules or carriers (such as positively charged polymers like poly(ethylene-imine) or PEI), which are themselves electrostatically coupled to the oxide layer. This is more likely the case when the charged entities are negatively charged. In both cases, this type of coupling allows for stable immobilization of the charged entities on the electrode surface while also permitting their release upon application of an electrical field. The strength of the electrostatic coupling can be influenced by factors such as the charge density of the entities, the surface, and any intermediary molecules, as well as the ionic strength of the surrounding medium. This versatility in coupling mechanisms enables the system to accommodate a wide range of charged entities and optimize their attachment and release for effective electroporation.

As used herein, and unless otherwise specified, the term *"negatively charged entities"* refers to molecules, particles, or complexes that carry a net negative electrical charge under the conditions used in the invention. In the context of this invention, negatively charged entities typically include, but are not limited to:
1. Nucleic acids: such as DNA (including plasmid DNA, linear DNA, and oligonucleotides), RNA (including mRNA, siRNA, and guide RNAs for CRISPR-Cas systems), and their synthetic analogues.
2. Proteins and peptides: that have a net negative charge at physiological pH due to an abundance of acidic amino acids (aspartic acid and glutamic acid) over basic amino acids.
3. Certain nanoparticles or carriers: that have been functionalized to carry a net negative charge.
4. Negatively charged small molecules: including certain drugs, dyes, or other bioactive compounds.
These negatively charged entities can interact electrostatically with positively charged intermediary molecules (carriers), allowing for their immobilization on the electrode surface. The nature and strength of the negative charge can influence the efficiency of both the electrostatic coupling to the electrode surface and the subsequent electroporation-mediated delivery into cells.

As used herein, and unless otherwise specified, the term "positively charged carriers (190)" refers to entities that carry a positive electric charge and can be used to electrostatically couple negatively charged entities to the oxide top layer. Examples of positively charged carriers include polymers (e.g., poly(ethylene-imine) (PEI) molecules, poly-lysine), and preferably branched polymers.

As used herein, and unless otherwise specified, the term *"entities differing in their characteristics"* refers to distinct entities that vary in one or more properties relevant to their interaction with or effect on biological cells. Examples of different characteristics include, but are not limited to, variations in chemical structure, sequence, size, charge, binding affinity, biological activity, or intended cellular target. In the context of nucleic acids, different characteristics may include variations in sequence, length, modifications, or encoded products. For proteins, different characteristics may include variations in amino acid sequence, post-translational modifications, or structural conformations. The term encompasses any set of entities where each member possesses at least one feature that distinguishes it from the others in the set, in a way that may produce different effects when interacting with biological cells.

As used herein, and unless otherwise specified, the phrase *"charged entities (10) differing in their characteristics are electrostatically coupled to distinct electrodes (140) of the microelectrode array (130)"* refers to the arrangement and immobilization of various charged entities differing in their characteristics on separate and individually addressable electrodes within the microelectrode array. This configuration allows for:
1. Spatial separation: Different charged entities can be physically isolated from each other by being attached to separate electrodes, preventing mixing or crosscontamination.
2. Multiplexed screening: Multiple distinct charged entities can be tested simultaneously on a single microelectrode array, enabling high-throughput experimentation.
3. Controlled release: The individual addressability of the electrodes allows for selective release and delivery of specific charged entities at chosen locations and times.
4. Diverse library testing: A wide range of charged entities with varying characteristics (such as different DNA sequences, RNA types, proteins, or other molecules) can be arranged and tested on the same device.
5. Correlation of effects with location: The known position of each unique charged entity on the array allows for easy correlation between the observed cellular effects and the specific entity responsible.

This arrangement is advantageous to enable the high-throughput screening capabilities of embodiments of the invention, allowing for the simultaneous evaluation of numerous distinct charged entities in a spatially resolved manner, thereby accelerating the discovery and optimization processes in various fields such as gene therapy, drug discovery, and biotechnology.

As used herein, and unless otherwise specified, the phrase "Providing a biological cellular material over the microelectrode array" refers to the placement of biological cells in a position above the microelectrode array, either in direct physical contact with the array surface or suspended at a distance above it. This placement can be achieved through various methods, including but not limited to: directly seeding cells onto the array surface and allowing them to adhere, introducing a cell suspension above the array without waiting for adhesion, or using a porous membrane or other spacing mechanism to maintain cells at a defined distance above the array surface. In the case of direct contact, cells may be allowed time to attach and spread on the array surface. In the case of suspension, cells may be maintained in a fluid medium above the array or supported by a porous membrane without direct contact with the electrode surface. The choice between contact and non-contact configurations may depend on factors such as cell type sensitivity, desired delivery efficiency, and spatial resolution requirements.

As used herein, and unless otherwise specified, the phrase "biological cellular material (30) in physical contact with said charged entities (10)" refers to the spatial arrangement where living cells or tissues are placed directly onto or immediately adjacent to the surface of the microelectrode array where the charged entities are electrostatically coupled. This configuration is characterized by:
1. Direct proximity: The biological cellular material is positioned such that there is no significant gap or intermediate layer between the cells and the electrostatically coupled charged entities on the electrode surface.
2. Cellular adhesion: The cells may adhere to the electrode surface, forming a layer that is in intimate contact with the charged entities.
3. Microenvironment interaction: The cells are exposed to and can interact with the local microenvironment created by the presence of the charged entities on the electrode surface.
4. Preparation for electroporation: This arrangement ensures that when an electrical field is applied, the cells are in the optimal position for the charged entities to be released and delivered into them through the temporarily permeabilized cell membranes.
5. Spatial correspondence: Each group of cells is in contact with specific charged entities coupled to distinct electrodes, allowing for location-specific delivery and effects.
6. Minimized diffusion: The close proximity minimizes the distance that released charged entities need to travel to enter the cells, potentially improving the efficiency of delivery.

This physical contact is advantageous for the effective operation of the device, as it ensures that when electroporation is induced, the charged entities are immediately available to enter the cells, maximizing the efficiency and specificity of the delivery process. It also allows for precise spatial control over which cells receive which charged entities, enabling high-resolution screening and analysis of the effects of different charged entities on cellular behavior.

As used herein, and unless otherwise specified, the term "electrical field (480)" refers to an electric field that is applied to one or more electrodes of the microelectrode array to cause electroporation of the biological cells and facilitate delivery of the charged entities into the cells.
As used herein, and unless otherwise specified, the phrase "*so as to release said charged entities (10) from the top layer (170)"* refers to the process by which the charged entities are detached or mobilized from the oxide top layer of the electrodes upon application of an electrical field. This release process can occur in two main scenarios:
Direct release: When charged entities are directly electrostatically coupled to the oxide top layer.
Indirect release: When negatively charged entities are electrostatically attached to positively charged carriers, which are themselves directly attached to the oxide top layer.
In both scenarios, the release process is characterized by:
a) Electrical field-induced detachment: The application of an electrical field disrupts the electrostatic interactions, causing the entities (or entity-carrier complexes) to become mobile.
b) Controlled release: The strength and duration of the applied electrical field can be modulated to control the extent and timing of the release.
c) Localized effect: Due to the individually addressable nature of the electrodes, the release can be spatially controlled.
d) Preservation of entity integrity: The release process is designed to mobilize the charged entities without damaging or altering their structure or function.
e) Transition to solution: Upon release, the charged entities or entity-carrier complexes transition from being surface-bound to being in solution or suspension in the medium surrounding the cells.
f) Preparation for cellular uptake: The release is timed to coincide with the temporary permeabilization of cell membranes during electroporation.
g) Potential for sequential or pulsed release: The release can be achieved in a single event or through multiple pulses.
In the case of indirect release (scenario 2):
h) Carrier behavior: The positively charged carriers may remain attached to the oxide layer, detach along with the negatively charged entities, or exhibit a combination of these behaviors depending on the strength of their electrostatic interactions and the applied electrical field.

This release step, whether direct or indirect, is advantageous for transitioning the charged entities from their immobilized state to a mobile state where they can be delivered into target cells through electroporation. The ability to control this release process spatially and temporally, and to accommodate different attachment scenarios, contributes to the precision, versatility, and efficiency of the delivery system described in the invention.

As used herein, and unless otherwise specified, the phrase *"detecting the effect"* refers to the process of observing, measuring, or quantifying the changes induced in the biological cells by the delivered entities, specifically using methods that are compatible with cells remaining on the substrate. This detection is preferably primarily performed through noninvasive or minimally invasive techniques that do not require removal or destruction of the cells from the microelectrode array. Examples include, but are not limited to, live-cell fluorescence microscopy, e.g., to visualize protein expression or cellular localization, label-free imaging techniques such as phase contrast or holographic microscopy, impedance measurements using a microelectrode array to assess cellular properties, bioluminescence imaging for reporter gene expression, and fluorescence-based assays for metabolic activity or membrane integrity that can be performed in situ. The detection may also involve the use of cell-permeable dyes or sensors that can be added to the culture medium. Techniques that require removal of cells from the substrate, such as flow cytometry, or that involve cell lysis, such as Western blotting or RT-qPCR, are preferably excluded from this definition in the context of this invention. The detection process is preferably designed to be high-throughput, allowing for the simultaneous assessment of effects across multiple locations on the substrate, and may involve real-time monitoring or defined time-point measurements to capture the range of cellular responses to the delivered entities.

As used herein, and unless otherwise specified, the term "dataset (360)" refers to a collection of data that includes information about the characteristics of the charged entities and the effects detected in the biological cells after electroporation with those charged entities.

As used herein, and unless otherwise specified, the phrase *"designing new entities based on the analysis of the dataset"* refers to the process of using the information gathered from previous screening rounds to inform the creation of new entities for subsequent testing. These new entities may be created virtually. These entities may also be synthesized. This process may involve interpreting the correlations between entity characteristics and their observed effects on biological cells, as captured in the dataset, to generate hypotheses about which features contribute to desired outcomes. The design process may employ various computational methods, including but not limited to, machine learning algorithms, statistical modeling, structure-activity relationship analyses, or rational design approaches. For nucleic acids, this might involve modifying sequences, altering structural elements, or introducing chemical modifications. For proteins, it could include amino acid substitutions, domain swapping, or changes in post-translational modifications. The goal is to iteratively refine and optimize the entities based on empirical data, potentially leading to entities with enhanced efficacy, reduced off-target effects, or novel functionalities. This design process is integral to the iterative nature of embodiments of the screening method, allowing for the continuous improvement and evolution of the entities being tested.

As used herein, and unless otherwise specified, the term *"control unit"* refers to a hardware or an integrated hardware and software system configured to pilot the device so that it performs steps b and c of the method according to any embodiment of the first aspect. It may be designed to manage, coordinate, and automate the various components and processes of the screening system. The control unit may be configured for orchestrating a sequence of operations comprising one or more of the following: controlling the application of the transfection activation field to specific locations on the substrate, managing the timing and parameters of entity delivery, coordinating detection systems, collecting and processing data, and adjusting experimental parameters in real-time based on feedback. It may incorporate microprocessors, field-programmable gate arrays (FPGAs), or other computational hardware, along with specialized software algorithms for data analysis, machine learning, and process optimization. The control unit may interface with the means for applying a transfection activation field, detection systems, and any auxiliary equipment, enabling precise temporal and spatial control over the screening process. It may also manage data storage, organization, and retrieval, facilitating the creation and analysis of the dataset that maps entity characteristics to observed effects. In advanced implementations, the control unit may incorporate adaptive learning algorithms to optimize screening parameters based on accumulated data, enhancing the efficiency and effectiveness of the screening process over time. The control unit is advantageous to achieve high-throughput, automatization of the screening system, minimizing the need for manual intervention, and ensuring reproducibility across experiments.

As used herein, and unless otherwise specified, the term *"UV-ozone and*/*or wet cleaning"* refers to surface preparation techniques used to clean and activate the substrate, particularly the microelectrode array, prior to the immobilization of entities. UV-ozone cleaning involves exposing the substrate surface to ultraviolet light in an oxygen-rich environment, generating highly reactive ozone molecules that oxidize and remove organic contaminants from the surface. This process also activates the surface by creating oxygencontaining functional groups, which can enhance subsequent binding of entities or other molecules. Wet cleaning, in the present context, refers to the use of liquid chemical solutions to remove contaminants and prepare the surface. This may include treatments with acids, bases, oxidizers, or organic solvents, followed by thorough rinsing, typically with pure water. The wet cleaning agents is preferably selected to be compatible with the electrode materials and to avoid damage to any underlying electronics. These cleaning methods may be used individually or in combination, depending on the specific requirements of the substrate and the entities to be immobilized. The cleaning process is advantageous for ensuring consistent and efficient immobilization of entities across the array, as well as for maintaining the performance and reliability of the microelectrodes. Unlike more aggressive cleaning methods such as oxygen plasma, which may damage sensitive CMOS-compatible electrode materials or underlying electronics, UV-ozone and appropriate wet cleaning protocols are designed to effectively prepare the surface while preserving the integrity of the microelectrode array.

As used herein, and unless otherwise specified, the phrase "means adapted to execute one or more steps, preferably all steps, of the method" refers to the components, devices, instruments, and/or software systems that are designed, configured, or programmed to perform the various operations described in the method claims. These means may include, but are not limited to:
Delivery unit: A component or system designed to deliver coupling means and/or charged entities to the microelectrode array. This may include:
   a) Liquid handling systems for precise dispensing of solutions
   b) Spotting or arraying tools for spatial patterning of entities on the array
   c) Fluidic systems for controlled introduction of materials
Electrical stimulation system: Equipment capable of applying controlled electrical fields to the electrodes, including:
   a) Voltage or current generators
   b) Pulse generators for creating specific waveforms
   c) Multiplexing systems for addressing individual electrodes

Cell culture support systems: Components that maintain appropriate conditions for biological cellular material, such as:
a) Temperature control systems
b) Gas regulation systems for maintaining proper CO2 levels
c) Humidity control systems

Detection and analysis systems: Equipment for observing and quantifying the effects of the charged entities on the biological cells, which may include:
a) Imaging systems (e.g., fluorescence microscopes, high-content imaging platforms)
b) Spectrophotometers or plate readers for quantitative measurements
c) Flow cytometers for cell analysis

Data acquisition and processing systems: Hardware and software for collecting, storing, and analyzing data generated during the method, including:
a) Computers or dedicated processing units
b) Data storage devices
c) Specialized software for data analysis and visualization

Control and automation systems: Components that coordinate and manage the operation of other systems, such as:
a) Programmable logic controllers (PLCs)
b) Robotics for automated sample handling
c) Software for experiment scheduling and process control

Biosafety equipment: Systems to ensure safe handling of biological materials, including:
a) Laminar flow hoods or biosafety cabinets
b) Sterilization equipment

User interface: Devices or software that allow users to input parameters, monitor processes, and review results, such as:
a) Touchscreen displays
b) Computer terminals with specialized software

Feedback and optimization systems: Components that can adjust parameters based on real-time measurements or results, including:
a) Sensors for monitoring cell membrane permeability
b) Adaptive control algorithms for optimizing electroporation parameters

These means are designed to work together to execute the steps of the method in a coordinated, efficient, and reproducible manner. The phrase "preferably all steps" indicates that while the system may be capable of performing individual steps separately, it is ideally configured to carry out the entire method from start to finish. This integrated approach allows for high-throughput screening, precise control over experimental conditions, and comprehensive data collection and analysis, all of which are crucial for the effective implementation of the invention.

The invention will now be described by a detailed description of several embodiments of the invention. It is clear that other embodiments of the invention can be configured according to the knowledge of persons skilled in the art without departing from the technical teaching of the invention, the invention being limited only by the terms of the appended claims.

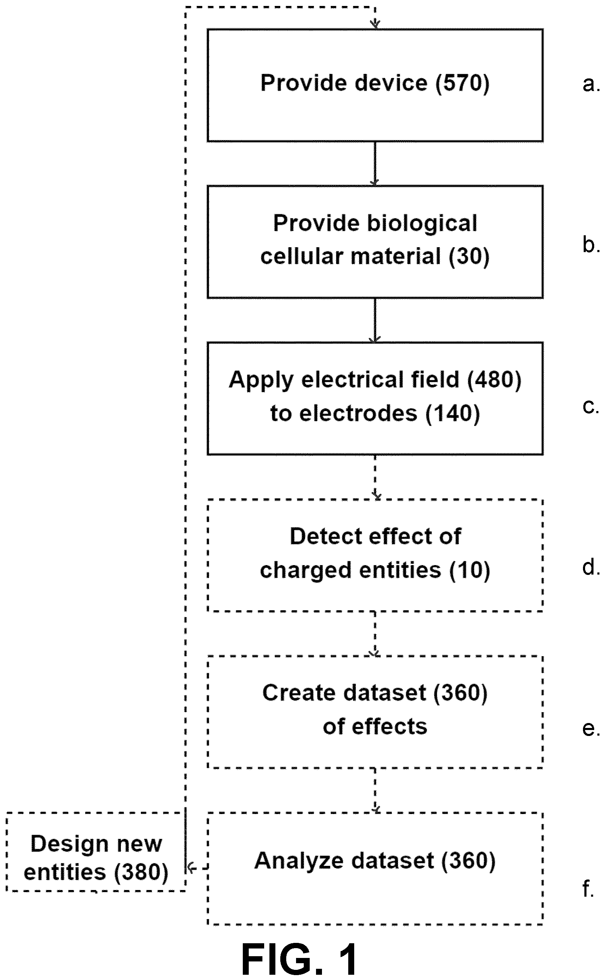

We now refer to Fig. 1, which illustrates a flowchart of a method for delivering charged entities (10) through electroporation into biological cells (20) of a biological cellular material (30) according to embodiments of the present invention. The method comprises a first step of a) providing a device (570) having a microelectrode array (130) with individually addressable electrodes (140) made of an electrically-conductive CMOS-compatible material (160) having an oxide top layer (170). Charged entities (10) are electrostatically coupled to the oxide top layer (170). In the second step b), a biological cellular material (30) is provided in physical contact with the charged entities (10). In the third step c), an electrical field (480) is applied to one or more of the electrodes (140) to release the charged entities (10) from the top layer (170) and allow the charged entities (10) to be delivered into biological cells (20) of the biological cellular material (30). The method may further include a fourth step d) of detecting an effect caused in the biological cells (20) by the delivered charged entities (10). The method may further include a fifth step e), after step d), of creating a dataset (360) comprising said characteristics of said charged entities (10) associated with the effects detected for said charged entities (10). Next, the method may comprise a sixth step f) of analyzing the dataset (360), e.g. using a machine learning algorithm (370), to correlate said effects with characteristics of the entities (10) associated with said effect. Next, the method may comprise a seventh step g) of designing new charged entities (380) based on the analysis of the dataset (360). Finally, the method my also comprise a step of repeating at least steps a to c, and optionally also steps d, e, f, and g while using the new charged entities (380) in step a.

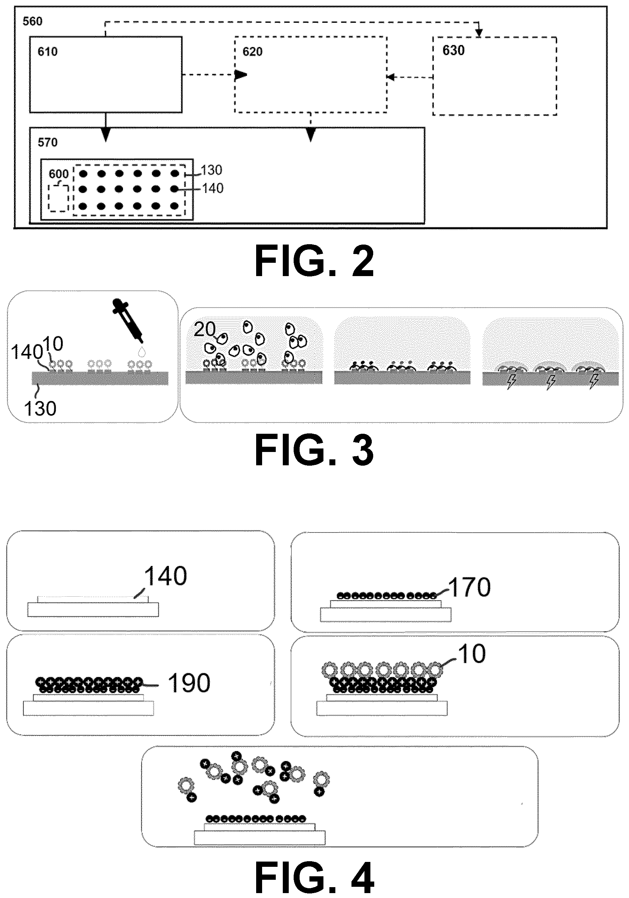

We now refer to Fig. 2, which shows a schematic diagram of a system (560) for high throughput screening of charged entities based on charged entity electrotransfer, i.e., delivery through electroporation, according to embodiments of the present invention. The system (560) comprises the device (570) having the microelectrode array (130) with individually addressable electrodes (140) made of the electrically-conductive CMOS-compatible material (160) with the oxide top layer (170). The system (560) further comprises means adapted to execute steps of the screening method, such as a delivery unit (620) for delivering coupling means and/or the charged entities (10) over the microelectrode array (130), a detector (600) for detecting effects on the biological cells (20), a control unit (610) for controlling operation of the system components, and means (630) for designing new entities.

We now refer to Fig. 3, which illustrates a schematic of a transfection screening workflow based on DNA immobilization on a microelectrode array (130) according to embodiments of the present invention. Different nucleic acids (80) are individually spotted and immobilized on the electrodes (140) of the microelectrode array (130). After cell seeding in bulk on the chip and cell adhesion to the electrode (140), sending electric pulses will both release the nucleic acids (80) from the electrodes (140) and electroporate the cells (20). During electroporation, cells (20) are permeable to nucleic acids (80) only during the submission of an electric field and will not pick-up free-floating nucleic acids (80) after the electroporation step. Cells (20) will be transfected only with the nucleic acid (80) on top of which they were adhering, thus achieving a spatially resolved transfection with multiple nucleic acids (80), the spatial resolution resulting from local spotting of nucleic acids (80). After transfection, the cells (20) will show a change of behavior corresponding to the nucleic acid (80) above which they grew. For instance, cells (20) may show a different fluorescent color depending on their location, that encodes the identity of the nucleic acid (80) that was delivered. In embodiments, thanks to the selective control of the electrodes on the microelectrode array, the spatial resolution of the DNA immobilization could be improved beyond the resolution of liquid dispensing tools, for instance by using electrical pulses to remove DNA from selected electrodes.

We now refer to Fig. 4, which illustrates a schematic of plasmid DNA immobilization on the native oxide layer (170) of a ruthenium or titanium nitride electrode (140) according to embodiments of the present invention. The plasmid DNA, which are negatively charged entities (180), are electrostatically attached to positively charged carriers (190), e.g., Poly(ethylene-imine) (PEI) molecules (200), themselves directly attached to the oxide top layer (170) of the electrode (140). The electrode (140) is made of an electrically-conductive CMOS-compatible material (160), such as ruthenium (270) or titanium nitride (260), having the native oxide top layer (170). Thanks to this non-covalent interaction, the plasmids can be released by applying weak electric pulses to the electrodes (140). In embodiments, the electrode materials may be different. CMOS-compatible electrode materials used may include Titanium (or Titanium Nitride), Platinum, Ruthenium, or Iridium. ITO is not compatible with CMOS, meaning ITO has issues with diffusion to substrate and is not be easily combinable with electronic circuits beneath the ITO electrode. In embodiments, the surface preparation may be different: The cleaning strategy used in prior art (oxygen plasma) may be avoided with the devices of the present invention, as it can damage the chip (overoxidation of the electrodes or electrically damaging the CMOS electronics). UV-Ozone and chemical cleaning may be looked at instead (see Fig. 3).

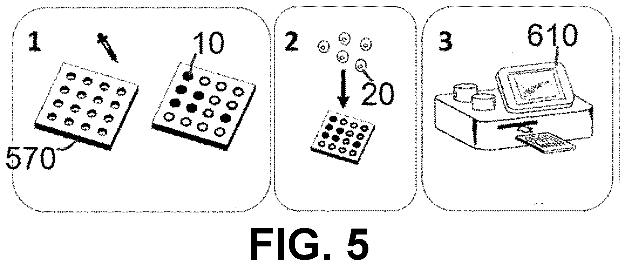

We now refer to Fig. 5, which illustrates a schematic of an example workflow for screening charged entities (10) using a pre-dispensed microelectrode array device (570) according to embodiments of the present invention. The pre-dispensing of charged entities (10) on chip will be performed in advance, in a fabrication facility for instance, before the device (570) is shipped to the user. Cell culture (and adding any reagents to perform a bioassay) will be the only step required from the user, as electroporation and analysis of results will be perform automatically by computer-controlled setups in a plug-and-play manner. Electroporation will be performed by plugging the microelectrode array (130) in a control setup (610) that will automatically apply an optimal electrical pulse to the cells (20), either preset or evaluated through electrical measurements done on the microelectrode array (130). A high content imaging tool (600) will automatically perform the analysis of the results. In embodiments, the electrodes may be connected in an array with individually addressable subjects.

### Example 1: High-throughput screening of nucleic acids using gene electrotransfer on microelectrode arrays

The aim of this experiment is to develop and demonstrate a high-throughput method for screening nucleic acids based on gene electrotransfer using microelectrode arrays (MEAs). This approach aims to overcome limitations of traditional bulk electroporation methods by enabling spatially-resolved transfection of multiple distinct nucleic acids on a single chip.

A microelectrode array chip is fabricated with electrodes made of ruthenium, which forms a native oxide layer on its surface. The chip contains 10,000 individually addressable microelectrodes in a 1 cm² area. To immobilize nucleic acids on the electrodes, the following procedure is used: First, the chip surface is cleaned using UV-ozone treatment for 10 minutes. Then, a 0.1% solution of poly(ethyleneimine) (PEI) in water is dispensed onto the chip surface and incubated for 30 minutes to allow electrostatic binding of the positively-charged PEI to the negatively-charged electrode oxide surface. After rinsing with deionized water, plasmid DNA encoding different fluorescent proteins is spotted onto individual electrodes using a microarray spotter, with each electrode receiving approximately 100 picoliters of a 100 ng/µL DNA solution. The chip is then air-dried and stored at 4°C until use.

To perform the transfection screening, HEK293 cells are seeded onto the MEA chip at a density of 100,000 cells/cm² in standard cell culture medium. The cells are allowed to adhere to the chip surface for 2 hours in a cell culture incubator. The chip is then connected to a custom electroporation control system. Electrical pulses (5 ms duration, 2 V amplitude) are applied to all electrodes simultaneously to trigger DNA release and cell electroporation. The cells are then returned to the incubator and cultured for 48 hours to allow for protein expression.

Fluorescence imaging of the entire chip is performed using an automated high-content imaging system. The results show distinct patches of fluorescence corresponding to the locations where different fluorescent protein-encoding plasmids had been spotted, indicating successful spatially-resolved transfection. Quantitative image analysis reveals that approximately 80% of cells directly above electrodes express the delivered genes, while minimal off-target transfection is observed. Cell viability assays show over 95% cell survival post-electroporation.

To demonstrate the high-throughput screening capability, a library of 1,000 different promoter sequences driving expression of a reporter gene is tested on a single chip. The entire workflow from cell seeding to data acquisition is completed within 3 days. Analysis of the fluorescence intensity data allows rapid identification of promoter sequences yielding high expression levels in the HEK293 cells.

This experiment successfully demonstrates a novel method for high-throughput nucleic acid screening using spatially-resolved electroporation on microelectrode arrays. The approach enables efficient testing of thousands of distinct nucleic acid constructs on a single chip with high transfection efficiency and cell viability. This platform has potential applications in fields such as gene therapy vector optimization, drug target discovery, and synthetic biology.

### Example 2: Screening of CRISPR guide RNAs using microelectrode array electroporation

This experiment aims to develop a high-throughput method for screening CRISPR guide RNAs (gRNAs) using microelectrode array (MEA) electroporation. The goal is to rapidly identify effective gRNAs for gene editing applications.

A microelectrode array chip is fabricated with 5,000 individually addressable platinum electrodes in a 0.5 cm² area. The chip surface is cleaned using a wet chemical process with piranha solution followed by deionized water rinses. A 0.5% solution of poly-L-lysine is then applied to the chip surface for 1 hour to create a positively charged layer for nucleic acid immobilization.

A library of 5,000 unique gRNA sequences targeting different sites in the human genome is synthesized. Each gRNA is complexed with Cas9 protein to form ribonucleoprotein (RNP) complexes. The RNP complexes are then spotted onto individual electrodes using a microarray spotter, with each electrode receiving approximately 50 picoliters of a 100 nM RNP solution. The chip is air-dried and stored at -20°C until use.

Human induced pluripotent stem cells (iPSCs) expressing a GFP reporter are seeded onto the MEA chip at a density of 200,000 cells/cm² in stem cell maintenance medium. After 12 hours of cell attachment, the chip is connected to an electroporation control system. Electrical pulses (2 ms duration, 1.5 V amplitude) are applied to all electrodes simultaneously to trigger RNP release and cell electroporation. The cells are then cultured for 72 hours to allow for gene editing to occur.
High-content imaging of the entire chip is performed to quantify GFP expression levels in cells above each electrode. Loss of GFP signal indicates successful gene editing by the delivered gRNA/Cas9 complexes. Image analysis reveals varying degrees of GFP knockout efficiency across the different gRNA sequences tested.

To validate the screening results, the top 50 gRNA sequences showing the highest GFP knockout efficiency are selected for further testing. These sequences are individually synthesized and tested in bulk cell populations using standard transfection methods. The results show a strong correlation between the MEA screening data and bulk validation experiments, confirming the reliability of the high-throughput screening approach.

The entire workflow from cell seeding to data acquisition is completed within 4 days, allowing rapid identification of highly effective gRNAs from a large library. This method demonstrates significant time and cost savings compared to traditional gRNA screening approaches.
In addition to GFP knockout efficiency, the MEA platform allows simultaneous assessment of cell viability and morphology changes induced by different gRNAs, providing valuable information on potential off-target effects.

This experiment successfully demonstrates the application of MEA-based electroporation for high-throughput screening of CRISPR guide RNAs. The approach enables efficient testing of thousands of gRNA sequences in stem cells with high spatial resolution and minimal reagent consumption. This platform has potential to accelerate the development of gene editing therapies and genomic engineering tools.

### Example 3: High-Throughput Screening of Protein Libraries for Improved Enzyme Activity

A library of mutant esterase enzymes is screened for improved catalytic activity using a microelectrode array (MEA) system. The MEA consists of 10,000 individually addressable electrodes fabricated from ruthenium, a CMOS-compatible material, each with a diameter of 30 µm and spaced 100 µm apart. The ruthenium electrodes possess a native oxide top layer that facilitates the electrostatic coupling of biomolecules.

The immobilization process begins with the preparation of the MEA surface. The array is cleaned using 10-minute UV-ozone exposure. This treatment enhances the oxide layer on the ruthenium electrodes and ensures a clean, hydrophilic surface. A solution of branched polyethyleneimine (PEl, 25 kDa) is prepared at 2 mg/mL in 100 mM sodium bicarbonate buffer (pH 8.4). Using a high-precision microarray spotter, 500 pL droplets of the PEI solution are deposited onto each electrode and allowed to dry in a humidity-controlled chamber (65% relative humidity) for 30 minutes. The MEA is then gently washed with deionized water and air-dried.

The protein library, consisting of 10,000 unique esterase variants, is prepared in a 96-well plate format. Each variant is expressed in E. coli and purified using affinity chromatography. The purified proteins are buffer-exchanged into a low-ionic strength buffer (10 mM HEPES, pH 7.0) to enhance electrostatic interactions. The protein concentration is adjusted to 50 µg/mL for each variant.

Using the microarray spotter, 500 pL of each esterase variant solution is precisely deposited onto individual PEI-coated electrodes. The spotting is performed in an environmentally controlled chamber at 18°C and 80% relative humidity to prevent droplet evaporation. The MEA is incubated for 2 hours at room temperature in a humidified chamber to allow for electrostatic coupling of the negatively charged proteins to the positively charged PEI layer. After incubation, the MEA is gently washed with phosphate-buffered saline (PBS) to remove any unbound protein and then air-dried in a laminar flow hood.

For the cell-based assay, a mammalian cell line (CHO-K1) is engineered to express a fluorogenic esterase substrate. The cells are cultured in DMEM/F-12 medium supplemented with 10% FBS and harvested using trypsin-EDTA. The cells are resuspended in a low-conductivity electroporation buffer (5 mM KCI, 15 mM MgCl₂, 120 mM trehalose, 25 mM HEPES, pH 7.4) at a density of 5 x 106 cells/mL.

The cell suspension is carefully pipetted onto the protein-immobilized MEA, ensuring uniform coverage. The cells are allowed to settle for 10 minutes at room temperature. The MEA is then connected to a multichannel electroporation system capable of individually addressing each electrode. Electroporation is performed using optimized parameters: three square-wave pulses of 1.5 kV/cm for 100 µs each, with a 1-second interval between pulses. This process releases the immobilized esterase variants from the electrodes and delivers them into the cells.

Immediately after electroporation, pre-warmed culture medium containing the fluorogenic esterase substrate is added to the MEA. The array is incubated in a live-cell imaging system maintained at 37°C and 5% CO₂. Fluorescence measurements are taken at 5-minute intervals for 4 hours to monitor the kinetics of substrate hydrolysis, which correlates with the activity of each esterase variant.

Data analysis is performed using custom image processing software that quantifies the fluorescence intensity associated with each electrode over time. The software generates kinetic profiles for each esterase variant, allowing for the identification of mutants with improved catalytic activity, altered substrate specificity, or enhanced stability.

### Example 4: High-Throughput Screening of Small Molecule Libraries on 3D Organoids

A library of 5,000 small molecule compounds is screened for their effects on liver organoid function using an advanced microelectrode array (MEA) system. The MEA consists of 5,000 individually addressable electrodes fabricated from iridium, a CMOS-compatible material, each with a diameter of 50 µm and spaced 150 µm apart. The iridium electrodes possess a native oxide top layer that facilitates the electrostatic coupling of the small molecules.

The MEA is designed with microwells surrounding each electrode to accommodate 3D organoids. Each microwell is 200 µm in diameter and 100 µm deep, created using a biocompatible photoresist material. This design allows for the culture of individual liver organoids in close proximity to the electrodes while maintaining their 3D structure.

The immobilization process begins with the preparation of the MEA surface. The array is cleaned using a 15-minute UV-ozone treatment, which enhances the oxide layer on the iridium electrodes and ensures a clean, hydrophilic surface. A solution of poly-L-lysine (PLL, 30-70 kDa) is prepared at 0.1 mg/mL in sterile water. Using a high-precision microarray spotter, 1 nL droplets of the PLL solution are deposited onto each electrode and allowed to dry in a humidity-controlled chamber (70% relative humidity) for 1 hour. The MEA is then gently washed with deionized water and air-dried.

The small molecule library, consisting of 5,000 unique compounds, is prepared in a 384-well plate format. These compounds have diverse chemical properties, including varying charges (positive or negative) at physiological pH. To accommodate this diversity and ensure efficient immobilization and release, the following strategy is employed:
For negatively charged molecules:
   The iridium electrodes are first coated with poly-L-lysine (PLL, 30-70 kDa) as described previously. This creates a positively charged layer that can electrostatically interact with negatively charged molecules or provide a surface for adsorption of neutral molecules.
For positively charged molecules:
   A subset of the electrodes is modified with a thin layer of polyacrylic acid (PAA) instead of PLL. This creates a negatively charged surface suitable for electrostatic interaction with positively charged molecules.

Each compound is dissolved in DMSO and diluted to a final concentration of 10 µM in a low-ionic strength buffer (5 mM HEPES, pH 7.2) with a final DMSO concentration of 0.1%. The pH of this buffer is chosen to maintain the expected charge state of the molecules as they would have under physiological conditions.

Using the microarray spotter, 500 pL of each small molecule solution is precisely deposited onto the appropriately prepared electrodes (PLL-coated for negative/neutral molecules, PAA-coated for positive molecules). The spotting is performed in an environmentally controlled chamber at 20°C and 85% relative humidity to prevent droplet evaporation.

The MEA is incubated for 30 minutes at room temperature in a sealed chamber to allow for electrostatic coupling or adsorption of the small molecules to the prepared electrode surfaces. After incubation, the MEA is gently washed with phosphate-buffered saline (PBS) to remove any unbound molecules and then air-dried in a laminar flow hood.

To validate the immobilization, a subset of electrodes is analyzed using surfaceenhanced Raman spectroscopy (SERS) to confirm the presence and stability of the immobilized compounds. Human liver organoids are derived from primary hepatocytes and cultured for 7 days to form 3D structures approximately 150 µm in diameter. The organoids are dissociated into smaller clusters of 30-50 µm using a mild enzymatic treatment. These clusters are resuspended in a low-conductivity electroporation buffer (5 mM KCI, 15 mM MgCl₂, 120 mM trehalose, 25 mM HEPES, pH 7.4) at a density of 1 × 10⁶ clusters/mL.

Using a computer-controlled micropipetting system, 2 nL droplets of the organoid cluster suspension are precisely deposited into each microwell of the MEA. The array is centrifuged at 100 x g for 1 minute to ensure the clusters settle into the microwells. The MEA is then incubated in a humidified chamber at 37°C and 5% CO₂ for 24 hours to allow the organoid clusters to re-aggregate and adhere to the microwells.

After incubation, the MEA is connected to a multichannel electroporation system capable of individually addressing each electrode. Electroporation is performed using optimized parameters: five square-wave pulses of 0.75 kV/cm for 50 µs each, with a 100 ms interval between pulses. This process releases the immobilized small molecules from the electrodes and delivers them into the organoids.

Immediately after electroporation, pre-warmed organoid culture medium is added to the MEA. The array is incubated in a live-cell imaging system maintained at 37°C and 5% CO₂. The system is equipped with a microfluidic perfusion setup that continuously supplies fresh medium and removes waste products, maintaining optimal conditions for the 3D organoids.

The effects of the small molecules on liver organoid function are assessed using multiple readouts:
1. Albumin secretion: Culture medium is sampled every 24 hours for 5 days and analyzed using a custom microfluidic ELISA system integrated with the MEA platform.
2. Cytochrome P450 activity: A luminescent CYP3A4 substrate is added to the medium on day 5, and luminescence is measured for each organoid using a high-sensitivity CCD camera.
3. Organoid morphology: Daily brightfield and fluorescence images are captured to assess changes in organoid size, shape, and viability (using a live/dead stain).
4. Gene expression: On day 5, the organoids are lysed in situ, and high-throughput qPCR is performed on a panel of 20 liver-specific genes using a integrated microfluidic qPCR system.

Data analysis is performed using machine learning algorithms that integrate all four readouts to identify small molecules that enhance liver organoid function, maintain long-term viability, or induce hepatocyte maturation. The top 50 hits are selected for further validation and dose-response studies.

### Example 5: High-throughput screening of nucleic acids using suspended cell electroporation

The experiment described in Example 1 is repeated with step c performed while the cells are in suspension above the electrodes rather than after allowing them to adhere to the surface. This can be achieved by introducing the cell suspension into the chamber immediately before electroporation without allowing the cells the time to adhere on the microelectrode array. Another more elaborate way to achieve this is with the incorporation of a porous membrane in the device above the microelectrode array surface and allowing the cells to naturally settle onto the porous membrane while remaining suspended above the electrode array before the performance of step c. To compensate for the increased distance between the electrodes and cells, the electrical pulse parameters are modified to deliver 8 ms pulses at 3 V amplitude, compared to the 5 ms, 2 V pulses used in the contact-based approach. All other aspects of the experiment remain unchanged. This method may further increase cell viability but may somewhat decrease transfection efficiency and/or special resolution.

It is to be understood that although preferred embodiments, specific constructions and configurations, as well as materials, have been discussed herein for devices according to the present invention, various changes or modifications in form and detail may be made without departing from the scope of this invention. For example, any formulas given above are merely representative of procedures that may be used. Functionality may be added or deleted from the block diagrams and operations may be interchanged among functional blocks. Steps may be added or deleted to methods described within the scope of the present invention.

## Claims

1. A device (570) for delivering charged entities (10) through electroporation into biological cells (20) of a biological cellular material (30), comprising: a microelectrode array (130) for supporting the biological cellular material (30), the microelectrode array (130) comprising individually addressable electrodes (140) made of an electrically-conductive CMOS-compatible material (160) having an oxide top layer (170), and the charged entities (10) electrostatically coupled to the oxide top layer (170).

2. The device (570) according to claim 1, wherein the charged entities (10) are negatively charged entities (180) electrostatically attached to positively charged carriers (190), themselves directly attached to the oxide top layer (170).

3. The device (570) of any one of the preceding claims, wherein charged entities (10) differing in their characteristics are electrostatically coupled to distinct electrodes (140) of the microelectrode array (130).

4. The device (570) of any one of the preceding claims, wherein the negatively-charged entities (180) are nucleic acids (80) or polypeptides.

5. A method for delivering charged entities (10) through electroporation into biological cells (20) of a biological cellular material (30), comprising: a. Providing a device (570) according to any one of the preceding claims, b. Providing a biological cellular material (30) over the microelectrode array (130), c. Applying an electrical field (480) to one or more of said electrodes (140) to which charged entities (10) are electrostatically coupled so as to release said charged entities (10) from the top layer (170) and allow said charged entities (10) to be delivered into said biological cells (20) of said biological cellular material (30).

6. The method according to claim 5, wherein in step a. said charged entities (10) differ in their characteristics and are electrostatically coupled to distinct electrodes (140) of the microelectrode array (130).

7. The method according to claim 5 or claim 6, further comprising a step d., after step c, of detecting the effect of the charged entities (10) on the biological cells (20) in which they have been delivered, and a step e., after step d., of creating a dataset (360) comprising said characteristics of said charged entities (10) associated with the effects detected for said charged entities (10).

8. The method according to claim 7, further comprising a step f. of analyzing the dataset (360), e.g. using a machine learning algorithm (370), to correlate said effects with characteristics of the entities (10) associated with said effect.

9. The method according to claim 8, further comprising a step g. of designing new charged entities (380) based on the analysis of the dataset (360).

10. The method according to claim 9, further comprising a step of repeating steps a to c while using the new charged entities (380) in step a.

11. The method according to any one of the preceding claims, wherein the electrical field (480) is individually applied to each of said electrodes (140) in a controlled manner, taking into account one or more cellular health factors, so as to enable electroporation while preventing cellular death directly caused by the electrical field (480).

12. A system (560) comprising the device (570) of any of claims 1 to 4 and further comprising means adapted to execute one or more steps, preferably all steps, of the method of any one of claims 5 to 11.

13. A computer program comprising instructions to cause the system (560) of claim 12 to execute the steps of the method of any one of claims 5 to 11.

14. A computer-readable medium having stored thereon the computer program of claim 13.

15. A kit for electroporating charged entities (10) into biological cells (20) of a biological cellular material (30), comprising: · a. A microelectrode array (130) for supporting the biological cellular material (30), the microelectrode array (130) comprising individually addressable electrodes (140) made of an electrically-conductive CMOS-compatible material (160) having an oxide top layer (170), · b. coupling means for electrostatically coupling charged entities (10) to said electrodes (140).
